(19) Europäisches Patentamt
European Patent Office
Office européen des brevets

(11) Publication number: **0 147 879**
**B1**

(12) # EUROPEAN PATENT SPECIFICATION

(45) Date of publication of patent specification: **16.12.87**

(51) Int. Cl.$^4$: **C 07 C 83/00**

(21) Application number: **84201735.2**

(22) Date of filing: **28.11.84**

(54) **Process for the preparation of a hydroxylamine.**

(30) Priority: **06.12.83 GB 8332555**
**26.01.84 NL 8400236**

(43) Date of publication of application:
**10.07.85 Bulletin 85/28**

(45) Publication of the grant of the patent:
**16.12.87 Bulletin 87/51**

(84) Designated Contracting States:
**AT BE CH DE FR GB IT LI LU NL SE**

(56) References cited:

**CHEMICAL ABSTRACTS, vol. 91, no. 7, August 13, 1979, page 669, abstract no. 56604w, COLUMBUS OHIO (US) & JP - A - 7924837**
**TETRAHEDRON, vol. 34, no. 2, 1978, pages 213-215, Pergamon Press, OXFORD (GB), I.D. ENTWISTLE et al.: "Rapid catalytic transfer reduction of aromatic nitro compounds to hydroxylamines"**

(73) Proprietor: **AKZO N.V.**
**Velperweg 76**
**NL-6824 BM Arnhem (NL)**

(72) Inventor: **Sharma, Ashutosh Haridatt**
**72 Elmsfield Avenue Norden**
**Rochdale Lancashire, OL11 5XN (GB)**
Inventor: **Hope, Peter**
**16, Lakeside Hollingworth Lake**
**Littleborough (GB)**

(74) Representative: **Sieders, René et al**
**P.O. Box 314**
**NL-6800 AH Arnhem (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 147 879 B1

**Description**

The present invention relates to a process for the preparation of a hydroxylamine of the formula

A: 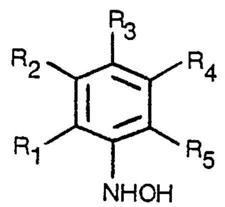 or B: RNHOH

In compound A, the groups $R_1$, $R_2$, $R_3$, $R_4$, $R_5$ each represent a hydrogen atom, a hydroxy group, a halogen atom, a linear or branched-chain alkyl group having 1 to 20 carbon atoms, an alkoxy group having 1 to 20 carbon atoms, a cyclopentyl group, a cyclohexyl group, a phenyl group, a phenylalkyl or alkylphenyl group, the alkyl group having 1 to 6 carbon atoms, or wherein two of the groups $R_1$, $R_2$, $R_3$, $R_4$ or $R_5$ attached to adjacent carbon atoms of the benzene ring together with the two carbon atoms of the benzene ring to which they are attached form a second benzene ring which is ortho-condensed with the first benzene ring. In compound B, the group R represents a linear or a branched-chain and/or cyclic alkyl group having 1 to 24 carbon atoms.

In so far as the Groups $R_1$—$R_5$ or R are hydrocarbon containing groups, they may have unsaturated bonds and may comprise substituents and groups such as amino, hydroxylamino, amido, cyano, hydroxy alkoxy, carboxyl, oxycarbonyl, carboxy, sulphoxide and sulphone. The groups $R_1$—$R_5$ or R may be combined to form part of a polymeric structure.

The aromatic hydroxylamine A can be obtained by hydrogenation of the corresponding nitro derivative in the presence of an inert solvent, a platinum catalyst and a tri- or penta valent organic phosphoric compound. Such a process is known from Chem. Abstracts, 91, (1979), 56604W, wherein is referred to Japanese Patent Publication No. 7 924 837.

Although a nitrogen containing base is formed as a byproduct of the hydrogenation, due to a too rapid reduction of the desired intermediate hydroxylamine, there was proposed for a long time to carry out the hydrogenation in the presence of an amount of an organic base to be added to the starting reaction mixture, like in eg. US Patent Specification 3 927 101. Herein it is stated that in order to obtain sufficiently high yields of hydroxylamines, the ratio by weight of the organic base to the nitro compound should be greater than 0.1:1, preferably between 0.5:1 and 5:1.

Further disclosed is that the use of the base in trace amounts does not make it possible to stop the hydrogenation at the hydroxylamine stage. After the hydrogenation reaction is completed, the organic base has to be removed. It has been observed that by using the above-mentioned high amounts of organic base, problems might arise upon isolating the hydroxylamine.

It has now been found that equivalent and even higher yields of hydroxylamine can be obtained with a process of the above type which is characterized in that the nitrogen-containing base is added to the starting reaction mixture in an amount of less than 10% by weight, calculated on the amount of the nitro derivative.

It has been found that if only use is made of the phosphorus compound or the nitrogen-containing base in an amount of less than 10% by weight, calculated on the amount of nitro derivative, the yields of isolated hydroxylamines are significantly reduced in comparison with those obtained with the process in the present invention.

It should be added that Japanese Patent Publication No. 30.096/82 discloses the use of trivalent phosphorus compounds in reactions of the present type. However, this publication does not mention the present combination with the nitrogen-containing base in the starting reaction mixture. Furthermore, U.S. 3 441 610 describes the catalytic hydrogenation of nitroalkanes to N-alkyl-hydroxylamines. Use is made of a recovered palladium catalyst and a cation of iron, nickel or cobalt in a two-phase liquid system of aqueous sulphuric acid and an immiscible organic solvent. In such an acidic medium the loss of catalyst will be considerable and the equipment may be subject to severe corrosion. These disadvantages are circumvented by the present invention.

The present invention relates to a process for the selective catalytic preparation of hydroxylamines with the structure A or B by selective catalytic hydrogenation of corresponding nitro derivatives.

The problem known for a long time as associated with the use of platinum-containing catalysts, is to prevent the nitro derivatives from being reduced to amines in the hydrogenation stage. The present process provides such a process without displaying the disadvantages of similar, well-known processes.

As organic phosphorus compound tri- or pentavalent compounds can be used. Trivalent phosphorus compounds are preferred. A class of suitable phosphorus compounds includes trivalent compounds having aryl or aryloxy groups which may be substituted or not.

Preferably the aryl and aryloxy groups are phenyl and phenyloxy groups. As Examples of phosphorus compounds of this class may be mentioned triphenylphosphite, dimethylphenylphosphite, triphenylphosphine, triphenylphosphonite, tri-p-chlorophenylphosphonite, tri-p-nitrophenylphosphonite and tricresylphosphonite.

Diphenylphosphite, di-p-chlorophenylphosphite, di-p-nitrophenylphosphite and di-p-methylphenylphosphite are also suitable phosphorus compounds.

A very suitable class includes phosphorus compounds having alkyl or alkoxy group(s) containing 1 to 20 carbon atoms. Especially preferred are trialkylphosphines and tri-alkylphosphites, with the alkyl group containing 1 to 20 carbon atoms. Examples thereof are: trimethylphosphine, tri-ethylphosphine, tri-isopropylphosphine, tri-butylphosphine, tri-octylphosphine, tri-octadecylphosphine, trimethylphosphite, tri-ethylphosphite, tri-isopropylphosphite, tributylphosphite, trihexylphosphite, triheptylphosphite, trioctylphosphite, trinonylphosphite, tridecylphosphite and trilaurylphosphite.

Most preferred thereof are compounds with alkyl groups having 1 to 6 carbon atoms, special preference being given to tri-ethylphosphite, tri-isopropylphosphite and tributylphosphite.

Further phosphorus compounds which can be used in the present process are: phosphorus trichloride, dimethylphosphochloridite, di-ethylphosphochloridite, and hexamethylphosphoroustri-amide. In the last two compounds the functions of nitrogen-containing base and phosphorus compound are combined. If such compounds are used, the amount of a further nitrogen base may be reduced.

Of course, also mixtures of the above phosphorus compound can be used. Generally, the reaction mixture should contain 0.1 to 5% by weight of the phosphorus compound, calculated on the amount of nitro derivative, preferably 0.2 to 2.0% and more particularly 0.3 to 1.0% by weight.

Suitable nitrogen bases are ammonia, monoalkylamines, dialkylamines, trialkylamines, monoalkanolamines, dialkanol amines, trialkanol amines, mono-, di- or tri-aryl amines, (poly)alkylene polyamines, pyrrolidone and piperidine substituted or not with 1 or 2 alkyl groups having 1 to 4 carbon atoms and pyridines substituted or not with one or more alkyl, amino, phenylalkylamino, phenylamino or pyrrolidone groups. The nitrogen base may contain primary, secondary and tertiary alkyl and/or alkanol groups. Generally, the above-mentioned unspecified alkyl and alkanol groups may contain 1 to 20, preferably 1 to 6 and most preferably 1 to 4 carbon atoms.

Examples of mono-, di- and trialkylamines are: methylamine, ethylamine, propylamine, butylamine, pentylamine, hexylamine, heptylamines, octylamine, nonylamine, octadecylamine, dimethylamine, diethylamine, dipropylamine, dipentylamine, dihexylamine, diheptylamine, dioctylamine, didecylamine, didodecylamine, dioctadecylamine, trimethylamine, triethylamine, tripropylamine, tri-isopropylamine, tributylamine, tripentylamine, trihexylamine, triheptylamine, tri-octylamine, tri-2-ethylhexylamine, tridecylamine and tri-octadecylamine.

Suitable mono-, di- and tri-alkanol amines are those having alkanol groups containing 1 to 20 and preferably 1 to 4 carbon atoms. Examples thereof are ethanolamine, propanolamine, butanolamine, diethanolamine and triethanolamine.

Suitable mono-, di- and tri-arylamines are N,N-diethyl-N-phenylamine, N-ethyl-N,N-diphenylamine, triphenylamine, tri-o-methylphenylamine, tri-m-methylphenylamine, tri-p-methylphenylamine, tribenzylamine, N-benzyl-N,N-dimethylamine, N-benzyl-N,N-diethylamine, N-benzyl-N,N-di-isopropylamine, N-benzyl-N,N-di-n-butylamine and N-benzyl-N,N-di-tert.-butylamine. Suitable (poly)alkylene polyamines are ethylenediamine, diethylenetriamine, triethylamine tetra-amine, tetra-ethylene pentamine, pentaethylene hexamine.

Pyrrolidine and piperidine as well as substituted pyrrolidines and piperidines containing 1 or 2 alkyl groups with 1 to 4 carbon atoms can also be used. Preferred examples thereof are pyrrolidine, piperidine and mono-, di-, tri- and tetramethylpyrrolidines and piperidines.

Most preferred are the pyridines substituted or not with one or more alkyl, amino, phenyl, alkylamino, phenylamino or pyrolidone groups, the alkyl groups containing preferably 1 to 6 carbon atoms.

Examples thereof are pyridine, N-methylpyridine, 2,6-dimethylpyridine, 4-methylethine, 4-aminopyridine, 4-dimethylaminopyridine, 4-di-ethylaminopyridine, 4-dipropylaminopyridine, 4-dibutylaminopyridine and 4-diphenylaminopyridine. Preference is given to the dialkylamino pyridines, particularly to 4-dimethylaminopyridine.

If course, also mixtures of the above nitrogen bases may be used. The reaction mixture should contain less than 10% by weight of nitrogen base, calculated on the amount of nitro derivative, general 0.05 to 5%, preferably 0.1 to 2% and more particularly 0.2 to 1% of the nitrogen base.

The nitro derivative starting material has the formula

C:

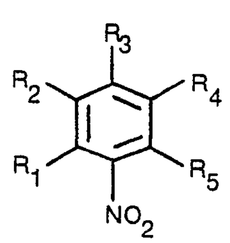

or

D: $RNO_2$

In compound C, the groups $R_1$, $R_2$, $R_3$, $R_4$ and $R_5$ each represent a hydrogen atom, a hydroxy group, a halogen atom, a linear or branched-chain alkyl group having 1 to 20, preferably 1 to 6, carbon atoms, an alkoxy group having 1 to 20, preferably 1 to 6, carbon atoms a cyclopentyl group, a cyclohexyl group, a phenyl group, a phenylalkyl or alkylphenyl group, the alkyl group having 1 to 6 carbon atoms, or wherein two of the groups $R_1$, $R_2$, $R_3$, $R_4$ or $R_5$ attached to adjacent carbon atoms of the benzene ring together with the two carbon atoms of the benzene ring which is ortho-condensed with the first benzene ring. In compound D, the group R represents a linear or a branched-chain and/or cyclic alkyl group having 1 to 24 carbon atoms.

In so far as the groups $R_1$—$R_5$ or R are hydrocarbon containing groups, they may have unsaturated bonds and may comprise substituents and groups such as amino, amido, nitro, nitroso, cyano, hydroxy, alkoxy, carbonyl, oxycarbonyl, carboxy, sulphoxide and sulphone. The substituents may be combined to form part of a polymer structure.

Examples of aromatic nitro-derivatives are nitrobenzene, o-nitrotoluene, m-nitrotoluene, p-nitrotoluene, p-isopropylnitrobenzene, m-butylnitrobenzene, 1,3-dimethyl-(2 or 4 or 5)nitrobenzene, 1,3,5-trimethyl-2-nitrobenzene, 4-nitro-biphenyl, para chloro nitrobenzene and (1 or 2 or 3)nitronaphthalene. Preference is given to nitrobenzene.

Examples of aliphatic nitro compounds are nitroethane, nitropropane, 2-methyl-2-nitropropane, 9-nitro-9-methyl-1,6-decadiene.

The catalyst should contain platinum and may or may not be deposited on a support. Suitable carrier materials are carbon blacks, alumina, silica, calcium carbonate, barium sulphate and the like.

When the platinum is on a carrier, the catalyst composition usually contains 0.1 to 20, preferably 1 to 10% of platinum. These catalysts can be prepared by methods well-known in the art. Generally, the reaction mixture contains 0.001 to 5%, preferably 0.01 to 1% by weight of platinum, calculated on the amount of nitro derivative.

The hydrogenation reaction is usually carried out in the presence of an inert solvent. There is no limitation on the solvent used, provided that it does not react with the hydroxylamine formed. Suitable solvents are water, lower alcohols such as methanol, ethanol, propanol, isopropanol, aromatic and aliphatic hydrocarbons such as toluene and hexane. Preference is given to a lower alcohol. Mixtures of the above solvents can also be used. The reaction mixture preferably contains 5 to 80% by weight of solvent.

The selective hydrogenation reaction can be carried out at 0° to 150°C, preferably between 5° and 50°C at a hydrogen pressure of 10 to 2000 kPa, preferably 100 to 500 kPa.

The present invention will be illustrated with the following examples.

## Example 1

A solution of nitrobenzene (24.6 g) in 80 ml of methanol is charged into a 600 ml autoclave; and 0.33 g of a catalyst based on platinum and carbon black containing 3% of platinum is added. 0.1 g of 4-dimethyl-amino pyridine and 0.25 g of tributylphosphite are then added to the autoclave. The autoclave is then sealed and deaerated using a vacuum pump. Next, hydrogenation is carried out at 10°C at 414 kPa from 3/4 hours after which time the theoretical amount of hydrogen was absorbed. After filtration of the Pt/C catalyst, the filtrate is evaporated by using a rotary evaporator under vacuum at 45°—55°C (bath temperature), after which the final traces of the solvent are removed using an oil pump. There are obtained 20.3 g of crude phenylhydroxylamine. The crude material is then treated with 75 ml of hexane, after which the mixture is well agitated and filtered. Finally, there are obtained 17.7 g of pure phenylhydroxylamine (yield 81.2%, m.p. 81°C, correct IR and NMR).

## Comparative Example A

Using the following amounts of starting materials and similar conditions as in Example 1, hydrogenation is carried out in the absence of 4-dimethylaminopyridine.

| | |
|---|---|
| Nitrobenzene | 12.3 g |
| Methanol | 40 ml |
| Tri-ethylphosphite | 0.322 g |
| 3% Pt/C | 0.400 g |
| Pressure of Hydrogen | 414 kPa |
| Temperature | 10°C |
| Time | 1 3/4 hours |

Finally, there are obtained 9.4 g of an oil which does not crystallize to give phenylhydroxylamine. The same results were obtained by using 0.25 g of tributylphosphite instead of triethylphosphite.

Comparative Example B

Using the following amounts of starting materials and similar conditions as in Example 1, hydrogenation is carried out in the absence of a trialkyl phosphite:

| | |
|---|---|
| Nitrobenzene | 24.6 g |
| Methanol | 80 ml |
| 4-dimethylaminopyridine | 2 g |
| 3% Pt/C | 0.4 g |
| Pressure of Hydrogen | 414 kPa |
| Temperature | 10°C |
| Time | 11/2 hours |

Finally, there are obtained 21 g of an oil which upon treatment with hexane (100 ml) followed by cooling to −30°C gives 10 g of crystalline phenylhydroxylamine. (Yield 45.9%, m.p. 80°C).

Example 2

The effects of adding different combinations of phosphorus compound and organic base was evaluated as follows: The general method was the same as in Example 1, except that the organic base and phosphorus component were varied as given in the following table. For this experiment the hydrogen pressure was maintained at 212 kPa and the final reaction mixture analysed, using gas liquid chromatography.

TABLE 1

| Triethylphosphite (TEP) | | Product composition (g) Phenylhydroxylamine (PHA) | | | |
|---|---|---|---|---|---|
| Level (TEP) | Base (g) | Nitrobenzene | Aniline | PHA | % Yield of PHA |
| 0.17 g | | 2.7 | 3.4 | 13.8 | 71 |
| 0.17 g | DMAP 0.05 | 0.4 | 2.2 | 18.8 | 87.7 |
| 0.17 g | DMAP 0.1 | 1.3 | 1.9 | 17.6 | 85.2 |
| 0.17 g | Pyridine 0.1 | 5.3 | 1.3 | 13.2 | 76 |
| Triphenylphosphine (TPP) | | | | | |
| Level TPP | Base | | | | |
| 0.17 g | | 4.4 | 1.1 | 14.2 | 78 |
| 0.17 g | DMAP 0.1 | | 2.3 | 18.6 | 85 |

DMAP = dimethylaminopyridine

Example 3

Using the following amounts of starting materials and similar conditions as in Example 1, hydrogenation is carried out as described before:

| | |
|---|---|
| 2-Nitrotoluene | 27.42 g |
| Methanol | 80 ml |
| 4—DMAP | 0.1 g |
| Tributylphosphite | 0.250 g |
| 3% Pt/C | 0.33 g |
| Pressure of Hydrogen | 212 kPa |
| Temperature | 32°C |
| Time | 4.0 hours |

There are obtained 21.8 g of an oil, which upon treatment with hexane (100 ml) followed by cooling to about −30°C gives 14.76 g of N-o-tolylhydroxylamine (Yield 60%, m.p. 44°C, correct IR and NMR spectrum).

Example 4

Using the following amounts of starting materials and similar conditions as in Example 1, hydrogenation is carried out as described before:

| | |
|---|---|
| 4-Nitrotoluene | 27.42 g |
| Methanol | 80 ml |
| 4—DMAP | 0.1 g |
| Tributylphosphite | 0.25 g |
| 3% Pt/C | 0.33 g |
| Pressure of Hydrogen | 212 kPa |
| Temperature | 25°C |
| Time | 1 hour |

There are obtained 24.5 g of an oil, which upon treatment with hexane (100 ml) followed by cooling to about −30°C gives 14.2 g of N-(p-tolyl)hydroxylamine (Yield 57.7%, m.p. 98°—99°C, correct IR and NMR spectrum).

Example 5

Using the following amounts of starting materials and similar conditions as in Example 1, hydrogenation is carried out as described before:

| | |
|---|---|
| p-chloro Nitrobenzene | 31.51 g |
| Methanol | 80 ml |
| 4—DMAP | 0.1 g |
| Tributylphosphite | 0.25 g |
| 3% Pt/C | 0.33 g |
| Pressure of Hydrogen | 414 kPa |
| Temperature | 25°C |
| Time | 2 hours |

There are obtained 25.2 g of an oil, which upon treatment with hexane (125 ml) followed by cooling to about −30°C gives 18.52 g of p-chloro phenylhydroxylamine (Yield 64.5%, m.p. 83°—84°C, correct IR and NMR spectrum).

Example 6

Using the following amounts of starting materials and similar conditions as in Example 1, hydrogenation was carried out as described before:

| | |
|---|---|
| 1-Nitropropane | 17.82 g |
| triethylamine | 0.10 g |
| triethylphosphite | 0.166 g |
| 3% Pt/C | 0.33 g |
| pressure of hydrogen | 345 kPa |
| temperature | 40°C |
| reaction time | about 5/6 hours |

The crude mixture was filtered and Pt/C removed for any subsequent use. The organic layer was treated with conc. HCl and cooled below ambient temperature where N-(1-propyl)hydroxylamine hydrochloride (17.84 g) was obtained in 80% yield.

Example 7

Using the following amounts of starting materials and similar conditions as given in Example 1, the hydrogenation of 1-nitropropane, 2-nitropropane, 2-methyl-2-nitropropane and 9-methyl-9-nitro-1,6-decadiene is carried out as described before:

| | |
|---|---|
| aliphatic nitro compound | 0.2 moles |
| 4-dimethylamino pyridine | 0.1 g |
| tributyl phosphite | 0.25 g |
| 3% Pt/C | 0.33 g |
| pressure of hydrogen | 414 kPa |
| temperature | 40°C |
| reaction time | about 3/4 hours |

The crude material is is treated with 75 ml of hexane, followed by vigorous agitation and subsequent filtration. The yields of N-(1-propyl)hydroxylamine, N-(2-propyl) hydroxylamine, N-(2-methylpropyl) hydroxylamine and 9-hydroxyl-amino-9-methyl-1,6-decadiene from 1-nitropropane, 2-nitropropane, 2-methyl-2-nitropropane and 9-methyl-9-nitro-1,6-decadiene are 60, 67, 67 and 60%, respectively.

**Claims**

1. A process for the preparation of a hydroxylamine of the formula

A: 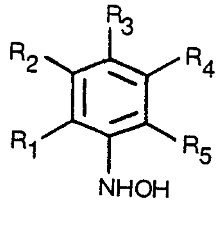 or B: RNHOH

in which $R_1$, $R_2$, $R_3$, $R_4$ and $R_5$ each represent a hydrogen atom, a hydroxy group, a halogen atom, a linear or branched-chain alkyl group having 1 to 20 carbon atoms, an alkoxy group having 1 to 20 carbon atoms, a

cyclopentyl group, a cyclohexyl group, a phenyl group, a phenylalkyl or alkylphenyl group, the alkyl group having 1 to 6 carbon atoms, or wherein two of the groups $R_1$, $R_2$, $R_3$, $R_4$ or $R_5$ attached to adjacent carbon atoms of the benzene ring together with the two carbon atoms of the benzene ring to which they are attached from a second benzene ring which is ortho-condensed with the first benzene ring, and R represents a linear or a branched-chain and/or cyclic alkyl group having 1 to 24 carbon atoms, by conversion of the corresponding nitro derivative by hydrogenation in the presence of an inert solvent, a platinum catalyst and a tri- or penta valent organic, phosphorus compound characterized in that a nitrogen containing base has been added to the starting reaction mixture in an amount of less than 10% by weight, calculated on the amount of the nitro derivative.

2. A process according to claim 1, characterized in that the hydroxylamine has the A structure.

3. A process according to claim 2, characterized in that the hydroxylamine has the B structure.

4. A process according to claim 1, 2 or 3 characterized in that the phosphorus compound contains at least one alkylgroup having 1 to 20 carbon atoms, or at least one aryl or aryloxy group.

5. A process according to claim 4, characterized in that the phosphorus compound is triphenylphosphine and/or triphenylphosphite.

6. A process according to claim 4, characterized in that the phosphorus compound is a trialkyl phosphine and/or a trialkyl phosphite.

7. A process according to claim 6, characterized in that the phosphorus compound is a trialkyl phosphite, with the alkyl groups containing 1 to 6 carbon atoms.

8. A process according to claim 7, characterized in that the phosphorus compound is triethylphosphite, tripropylphosphite and/or tributylphosphite.

9. A process according to one of the preceding claims, characterized in that the nitrogen-containing base is a di- or tri-alkyl amine, with the alkyl groups containing 1 to 6 carbon atoms.

10. A process according to claim 9, characterized in that the nitrogen containing base is triethylamine.

11. A process according to any one of the preceding claims 1 to 8, characterized in that the nitrogen-containing base is pyridine substituted or not with one or more alkyl, amino, phenyl, alkylamino, phenylamino or pyrrolidone groups, the alkyl groups containing 1 to 6 carbon atoms.

12. A process according to claim 11, characterized in that the nitrogen containing base is a dialkylaminopyridine.

13. A process according to claim 12, characterized in that the nitrogen containing base is a dimethylaminopyridine.

**Patentansprüche**

1. Verfahren zur Herstellung eines Hydroxylamins der Formeln

A: 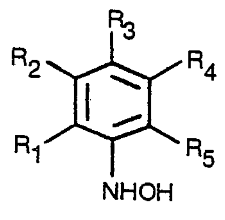 oder B: RNHOH

in der $R_1$, $R_2$, $R_3$, $R_4$ und $R_5$ jeweils ein Wasserstoffatom, eine Hydroxygruppe, ein Halogenatom, eine gerad- oder verzweigtkettige Alkylgruppe mit 1 bis 20 C-Atomen, eine Alkoxygruppe mit 1 bis 20 C-Atomen, eine Cyclopentylgruppe, eine Cyclohexylgruppe, eine Phenylgruppe, eine Phenylalkyl-oder Alkylphenylgruppe bedeuten, wobei die Alkylgruppe 1 bis 6 C-Atome hat, oder wobei zwei der Gruppen $R_1$, $R_2$, $R_3$, $R_4$ oder $R_5$, die an benachbarten C-Atomen des Benzolrings hängen, zusammen mit den zwei C-Atomen des Benzolrings, an dem sie hängen, einen zweiten Benzolring bilden, der orthoständig an den ersten Benzolring ankondensiert ist, und R eine gerad- oder verzweigtkettige und/oder cyclische Alkylgruppe mit 1 bis 24 C-Atomen bedeutet, durch Umwandlung des entsprechenden Nitroderivats durch Hydrierung in Gegenwart eines inerten Lösungsmittels, eines Platinkatalysators und einer drei- oder fünfwertigen organischen Phosphorverbindung, dadurch gekennzeichnet, dass der Startreaktionsmischung eine Stockstoff enthaltende Base in einem Anteil von weniger als 10 Gew.%, berechnet auf den Anteil des Nitroderivats, zugesetzt worden ist.

2. Verfahren nach Anspruch 1, dadurch gekennzeichnet, dass das Hydroxylamin die A-Struktur besitzt.

3. Verfahren nach Anspruch 1, dadurch gekennzeichnet, dass das Hydroxylamin die B-Struktur besitzt.

4. Verfahren nach Anspruch 1, 2 oder 3, dadurch gekennzeichnet, dass die Phosphorverbindung mindestens eine Alkylgruppe, die 1 bis 20 C-Atome enthält, oder mindestens eine Aryl- oder Aryloxygruppe aufweist.

5. Verfahren nach Anspruch 4, dadurch gekennzeichnet, dass die Phosphorverbindung Triphenylphosphin und/oder Triphenylphosphit ist.

6. Verfahren nach Anspruch 4, dadurch gekennzeichnet, dass die Phosphorverbindung ein Trialkylphosphin und/oder ein Trialkylphosphit ist.

7. Verfahren nach Anspruch 6, dadurch gekennzeichnet, dass die Phosphorverbindung ein Trialkylphosphit ist, dessen Alkylgruppen 1 bis 6 C-Atome enthalten.

8. Verfahren nach Anspruch 7, dadurch gekennzeichnet, dass die Phosphorverbindung Triethylphosphit, Tripropylphosphit und/oder Tributylphosphit ist.

9. Verfahren nach einem der vorangehenden Ansprüche, dadurch gekennzeichnet, dass die Stickstoff enthaltende Base ein Di- oder Trialkylamin ist, dessen Alkylgruppen 1 bis 6 C-Atome enthalten.

10. Verfahren nach Anspruch 9, dadurch gekennzeichnet, dass die Stickstoff enthaltende Base Triethylamin ist.

11. Verfahren nach einem der vorangehenden Ansprüche 1—8, dadurch gekennzeichnet, dass die Stickstoff enthaltende Base Pyridin ist, das gegebenenfalls mit einer oder mehreren Alkyl-, Amino-, Phenyl-, Alkylamino-, Phenylamino- oder Pyrrolidongruppen substituiert ist, wobei die Alkylgruppen 1 bis 6 C-Atome enthalten.

12. Verfahren nach Anspruch 11, dadurch gekennzeichnet, dass die Stickstoff enthaltende Base ein Dialkylaminopyridin ist.

13. Verfahren nach Anspruch 12, dadurch gekennzeichnet, dass die Stickstoff enthaltende Base ein Dimethylaminopyridin ist.

## Revendications

1. Procédé pour la fabrication d'une hydroxylamine de formule:

A: 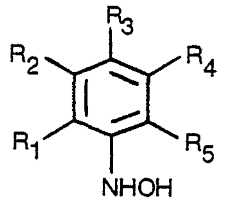 ou B: RNHOH

dans lesquelles $R_1$, $R_2$, $R_3$, $R_4$ et $R_5$ représentent chacun un atome d'hydrogène, un groupe hydroxy, un atome d'halogène ou un groupe alkyle à chaîne droite ou ramifiée en $C_1$—$C_{20}$, un groupe alcoxy en $C_1$—$C_{20}$, un groupe cyclopentyle, un groupe cyclohexyle, un groupe phényle, un groupe phénylalkyle ou alkylphényle dont le groupe alkyle a de 1 à 6 atomes de carbone, ou bien deux des groupes $R_1$, $R_2$, $R_3$, $R_4$ et $R_5$ pris ensemble avec les deux atomes de carbone voisins du noyau benzénique auxquels ils sont lié forment un second noyau benzénique qui est ortho-condensé avec le premier noyau benzénique, et R représente un groupe alkyle à chaîne droite ou ramifiée et/ou cyclique ayant de 1 à 24 atomes de carbone, par conversion du dérivé nitro correspondant par hydrogénation en présence d'un solvant inerte, d'un catalyseur au platine et d'un composé organique du phosphore tri- ou pentavalent, caractérisé en ce que l'on a ajouté au mélange de réaction de départ une base azotée en quantité de moins de 10% en poids, par rapport à la quantité du dérivé nitro.

2. Procédé selon la revendication 1, caractérisé en ce que l'hydroxylamine répond à la formule A.

3. Procédé selon la revendication 2, caractérisé en ce que l'hydroxylamine répond à la formule B.

4. Procédé selon la revendication 1, 2 ou 3, caractérisé en ce que le composé de phosphore contient au moins un groupe alkyle en $C_1$—$C_{20}$ ou au moins un groupe aryle ou aryloxy.

5. Procédé selon la revendication 4, caractérisé en ce que le composé de phosphore est la triphénylphosphine et/ou le phosphite de triphényle.

6. Procédé selon la revendication 4, caractérisé en ce que le composé de phosphore est une trialkylphosphine et/ou un phosphite de trialkyle.

7. Procédé selon la revendication 6, caractérisé en ce que le composé de phosphore est un phosphite de trialkyle dont les groupes alkyles contiennent de 1 à 6 atomes de carbone.

8. Procédé selon la revendication 7, caractérisé en ce que le composé de phosphore est le phosphite de triéthyle, le phosphite de tripropyle et/ou le phosphite de tributyle.

9. Procédé selon l'une quelconque des revendications précédentes, caractérisé en ce que la base azotée est une di- ou trialkylamine dont les groupes alkyles contiennent de 1 à 6 atomes de carbone.

10. Procédé selon la revendication 9, caractérisé en ce que la base azotée est la triéthylamine.

11. Procédé selon l'une quelconque des revendications précédentes 1 à 8, caractérisé en ce que la base azotée est la pyridine substituée ou non par un ou plusieurs groupes alkyles, amino, phényle, alkylamino, phénylamino ou pyrrolidone, les groupes alkyles contenant de 1 à 6 atomes de carbone.

12. Procédé selon la revendication 11, caractérisé en ce que la base azotée est une dialkylaminopyridine.

13. Procédé selon la revendication 12, caractérisé en ce que la base azotée est une diméthylaminopyridine.